# FASCICULE DE BREVET EUROPEEN

(11) **EP 0 560 968 B1**
(45) Date de publication et mention de la délivrance du brevet: **24.07.1996**
(21) Numéro de dépôt: 92921362.7
(22) Date de dépôt: 29.09.1992
(51) Int. Cl.: A61K 39/095, C07K 14/22

(54) **VACCIN CONTRE LES INFECTIONS A NEISSERIA MENINGITIDIS**
IMPFSTOFF GEGEN INFEKTIONEN DURCH NEISSERIA MENINGITIDIS
VACCINE FOR NEISSERIA MENINGITIDIS INFECTIONS

(30) Priorité: 03.10.1991 FR 9112177
(43) Date de publication de la demande: 22.09.1993
(73) Titulaire: PASTEUR MERIEUX SERUMS ET VACCINS, Société Anonyme :, F-69007 Lyon (FR)
(72) Inventeur: QUENTIN-MILLET, Marie-José, F-69100 Villeurbanne (FR)
(74) Mandataire: Bernasconi, Jean
(86) Numéro de dépôt international: FR9200905
(87) Numéro de publication internationale: WO9306861

(56) Documents cités:
- WO-A-87/02678
- WO-A-90/12591
- INFECTION AND IMMUNITY vol. 58, no. 9, Septembre 1990, WASHINGTON pages 2875 - 2881 NIRUPAMA B.B. ET AL 'expression of neisseria meningitidis iron-regulated outer membrane proteins, including a 70-kilodalton transferrin receptor, and their potential for use as vaccines'/

## Description

La présente invention a pour objet une composition pharmaceutique vaccinale destinée à la prévention des méningites causées par *Neisseria meningitidis*.

D'une manière générale, les méningites sont soit d'origine virale, soit d'origine bactérienne. Les bactéries principalement responsables sont : *N. meningitidis* et *Haemophilus influenzae*, respectivement impliquées dans environ 40 et 50 % des cas de méningites bactériennes.

On dénombre en France, environ 600 à 800 cas par an de méningites à *N. meningitidis*. Aux Etats-Unis, le nombre de cas s'élève à environ 2 500 à 3 000 par an.

L'espèce *N. meningitidis* est sub-divisée en sérogroupes selon la nature des polysaccharides capsulaires. Bien qu'il existe une douzaine de sérogroupes, 90 % des cas de méningites sont attribuables à 3 sérogroupes : A, B et C.

Il existe des vaccins efficaces à base de polysaccharides capsulaires pour prévenir les méningites à *N. meningitidis* sérogroupes A et C. Ces polysaccharides tels quels ne sont que peu ou pas immunogéniques chez les enfants de moins de 2 ans et n'induisent pas de mémoire immunitaire. Toutefois, ces inconvénients peuvent être surmontés en conjuguant ces polysaccharides à une protéine porteuse.

Par contre, le polysaccharide de *N. meningitidis* groupe B n'est pas ou peu immunogène chez l'homme, qu'il soit sous forme conjuguée ou non. Ainsi, il apparait hautement souhaitable de rechercher un vaccin à l'encontre des méningites induites par *N. meningitidis* notamment du sérogroupe B autre qu'un vaccin à base de polysaccharide.

A cette fin, différentes protéines de la membrane externe de *N. meningitidis* ont déjà été proposées. Il s'agit en particulier du récepteur membranaire de la transferrine humaine.

D'une manière générale, la grande majorité des bactéries ont besoin de fer pour leur croissance et elles ont développé des systèmes spécifiques d'acquisition de ce métal. En ce qui concerne notamment *N. meningitidis* qui est un pathogène strict de l'homme, le fer ne peut être prélevé qu'à partir des protéines humaines de transport du fer telles que la transferrine et la lactoferrine puisque la quantité de fer sous forme libre est négligable chez l'homme (de l'ordre de : 10⁻¹⁸ M), en tout cas insuffisante pour permettre la croissance bactérienne.

Ainsi, *N. meningitidis* possède un récepteur de la transferrine humaine et un récepteur de la lactoferrine humaine qui lui permettent de fixer ces protéines chélatrices du fer et de capter par la suite le fer nécessaire à sa croissance.

Le récepteur de la transferrine de la souche *N. meningitidis* B16B6 a été purifié par Schryvers et al (WO 90/12591) à partir d'un extrait membranaire. Cette protéine telle que purifiée apparaît essentiellement constituée de 2 types de polypeptides : un polypeptide d'un poids moléculaire apparent élevé de 100 kD et un polypeptide d'un poids moléculaire apparent moindre d'environ 70 kD, tels que révélés après électrophorèse sur gel de polyacrylamide en présence de SDS.

Le produit de la purification notamment mise en oeuvre par Schryvers est appelé, par définition arbitraire et pour les besoins de la présente demande de brevet, récepteur de la transferrine et les polypeptides le constituant, des sous-unités. Dans la suite du texte, les sous-unités de poids moléculaire élevé et de poids moléculaire moindre sont respectivement appelées Tbp1 et Tbp2.

On a maintenant trouvé qu'il existait au moins 2 types de souches qui diffèrent par la constitution de leurs récepteurs de la transferrine respectifs. Ceci a été mis en évidence en étudiant des extraits membranaires de plusieurs dizaines de souches de *N. meningitidis* d'origines variées. Ces extraits membranaires ont tout d'abord été soumis à une électrophorèse sur gel de SDS-PAGE, puis électrotransferés sur feuilles de nitrocellulose. Ces feuilles de nitrocellulose ont été incubées :
a) en présence d'un antisérum de lapin dirigé contre le récepteur de la transferrine purifié à partir de la souche *N. meningitidis* B16B6, aussi appellée 2394 ;
b) en présence d'un antisérum de lapin dirigé contre le récepteur de la transferrine purifié à partir de la souche *N. meningitidis* 2169 ; ou
c) en présence de la transferrine humaine conjuguée à la peroxydase.

En ce qui concerne a) et b), la reconnaissance des sous-unités du récepteur de la transferrine est révélée par addition d'un anticorps anti-immunoglobulines de lapin couplé à la peroxydase, puis par addition du substrat de cette enzyme.

Les tableaux I et II ci-après dessous indiquent le profil de certaines souches représentatives tel qu'il apparait sur gel de SDS-PAGE à 7,5 % polyacrylamide; les bandes sont caractérisées par leur poids moléculaire apparent exprimé en kilodaltons (kD) :

| | Souches | | |
|---|---|---|---|
| Tableau I | 2394 (B; 2a;P1.2:L2,3) 2228 (B; nd) 2170 (B; 2a:P1.2:L3) | 2234 (Y;nd) 2154 (C; nd) 2448 (B; nd) | 550 (C; 2a:) 179 (C; 2a:P1.2) |
| Détection avec l'antisérum anti-récepteur 2394 | 93 | 93 | 99 |
| | 68 | 69 | 69 |
| Détection avec l'antisérum anti-récepteur 2169 | 93 | 93 | 99 |
| Détection avec la transferrine peroxydase | 68 | 69 | 69 |
| N.B. : Entre parenthèse sont indiqués dans l'ordre le sérogroupe, le sérotype, le sous-type et l'immunotype. | | | |

| | Souches | | | | | | | | |
|---|---|---|---|---|---|---|---|---|---|
| Tableau II | 2169 (B:9:P1.9) | 1000 (B:nd) | 1604 (B:nd) | 132 (C:15: P1.16) | 1001 (A:4: P1.9) | 876 (B:19: P1.6) | 1951 (A:nd) | 2449 (B:nd) | 867 (B:2b:P1.2) |
| Détection avec l'antisérum anti-récepteur 2394 | 96 | 98 | 98 | 98 | 98 | 96 | 94 | 94 | 93 |
| Détection avec l'antisérum anti-récepteur 2169 | 96 | 98 | 98 | 98 | 98 | 96 | 94 | 94 | 93 |
| | 87 | 85 | 83 | 81 | 79 | 88 | 87 | 85 | 85 |
| Détection avec la trans-ferrineperoxydase | 87 | 85 | 83 | 81 | 79 | 88 | 87 | 85 | 85 |
| N.B.: Entre parenthèse sont indiqués dans l'ordre le sérogroupe, le sérotype, le sous-type et l'immunotype. | | | | | | | | | |

Les résultats répertoriés dans les 2 premières lignes des tableaux montrent qu'il existe 2 types de souches :

Le premier type (Tableau I) correspond à des souches qui possèdent un récepteur dont les 2 sous-unités sont reconnues par l'antisérum anti-récepteur 2394 tandis que seule la sous-unité de haut poids moléculaire est reconnue par l'antisérum anti-récepteur 2169.

Le second type (Tableau II) correspond à des souches qui possèdent un récepteur dont les 2 sous-unités sont reconnues par l'antisérum anti-récepteur 2169 tandis que seule la sous-unité de haut poids moléculaire est reconnue par l'antisérum anti-récepteur 2394.

En conséquence, il existe une diversité antigénique au niveau de la sous-unité de moindre poids moléculaire. Cette diversité est toutefois restreinte puisqu'elle se résout en 2 grands types, contrairement à ce qui est suggéré par Griffiths et al, FEMS Microbiol. Lett. (1990) 69 : 31.

[Par ailleurs, on notera que quelque soit le type de souche, la sous-unité capable de se lier à la transferrine est toujours la sous-unité de moindre poids moléculaire (Tableaux A et B, troisième ligne des résultats).]

En vertu de ces constatations, il eut été tentant de conclure qu'un vaccin efficace à l'encontre de toutes les infections à *N. meningitidis* pouvait être constitué de manière suffisante, d'un récepteur de la transferrine ou uniquement de sa sous-unité de haut poids moléculaire, quelle que soit la souche d'origine du récepteur, puisque cette dernière est reconnue par les 2 types d'antisérums.

De manière surprenante, on a maintenant trouvé que tel n'était pas le cas dans la mesure où la sous-unité de haut poids moléculaire ne serait pas capable d'induire la production d'anticorps de type neutralisant. Seule la plus petite des 2 sous-unités du récepteur serait capable de remplir cette fonction. Puisque cette sous-unité de moindre poids moléculaire se caractérise par une variation antigénique significative du premier type au deuxième type de souche, un seul type de récepteur de la transferrine ne devrait pas être suffisant pour vacciner contre toutes les infections à *N. meningitidis.*

C'est pourquoi l'invention propose :
i) Une composition pharmaceutique vaccinale qui comprend à titre d'agents thérapeutiques au moins une première et une deuxième molécules capables de se lier à la transferrine humaine ; ladite première molécule ayant pour origine une première souche de *N. meningitidis* qui possède un récepteur de la transferrine humaine au moins constitué d'une sous-unité de haut poids moléculaire (Tbp1) et d'une sous-unité de poids moléculaire moindre (Tbp2) et dont la sous-unité de poids moléculaire moindre (Tbp2) est reconnue par un antisérum anti-récepteur de la souche *N. meningitidis* 2394 (récepteur 2394) et n'est pas reconnue par un antisérum anti-récepteur de la souche de *N. meningitidis* 2169 (récepteur 2169) ; et ladite deuxième molécule ayant pour origine une deuxième souche de *N. meningitidis* qui possède un récepteur de la transferrine humaine au moins constitué d'une sous-unité de haut poids moléculaire (Tbp1) et d'une sous-unité de poids moléculaire moindre (Tbp2) et dont la sous-unité de poids moléculaire moindre (Tbp2) est reconnue par un antisérum anti-récepteur 2169 et n'est pas reconnue par un antisérum anti-récepteur 2394 ;
ii) Un kit de vaccination contenant :
   a) Une composition pharmaceutique qui comprend à titre d'agent thérapeutique au moins une première molécule capable de se lier à la transferrine humaine ; ladite première molécule ayant pour origine une première souche de *N. meningitidis* qui possède un récepteur de la transferrine humaine au moins constitué d'une sous-unité de haut poids moléculaire et d'une sous-unité de poids moléculaire moindre et dont la sous-unité de poids moléculaire moindre est reconnue par un antisérum anti-récepteur de la souche *N. meningitidis* 2394 (récepteur 2394) et n'est pas reconnue par un antisérum anti-récepteur de la souche de *N. meningitidis* 2169 (récepteur 2169) ;
   b) Une composition pharmaceutique qui comprend à titre d'agent thérapeutique au moins une deuxième molécule capable de se lier à la transferrine humaine ; ladite deuxième molécule ayant pour origine une deuxième souche de *N. meningitidis* qui possède un récepteur de la transferrine humaine au moins constitué d'une sous-unité de haut poids moléculaire et d'une sous-unité de poids moléculaire moindre et dont la sous-unité de poids moléculaire moindre est reconnue par un antisérum anti-récepteur 2169 et n'est pas reconnue par un antisérum anti-récepteur 2394. ; et
   c) Des instructions pour l'administration concomitante ou consécutive des compositions a) et b) ;
iii) L'usage thérapeutique combiné d'au moins une première et une deuxième molécules capables de se lier à la transferrine humaine ; ladite première molécule ayant pour origine une première souche de *N. meningitidis* qui possède un récepteur de la transferrine humaine au moins constitué d'une sous-unité de haut poids moléculaire et d'une sous-unité de poids moléculaire moindre et dont la sous-unité de poids moléculaire moindre est reconnue par un antisérum anti-récepteur de la souche *N. meningitidis* 2394 (récepteur 2394) et n'est pas reconnue par un antisérum anti-récepteur de la souche de *N. meningitidis* 2169 (récepteur 2169) ; et ladite deuxième molécule ayant pour origine une deuxième souche de *N. meningitidis* qui possède un récepteur de la transferrine humaine au moins constitué d'une sous-unité de haut poids moléculaire et d'une sous-unité de poids moléculaire moindre et dont la sous-unité de poids moléculaire moindre est reconnue par un antisérum anti-récepteur 2169 et n'est pas reconnue par un antisérum anti-récepteur 2394 ; et
iv) Une méthode de vaccination à l'encontre des infections à *N. meningitidis,* qui comprend l'acte d'administrer une quantité efficace d'un point de vue thérapeutique d'au moins une première et une deuxième molécules capables de se lier à la transferrine humaine, de manière concomitante ou consécutive, à un sujet ayant besoin d'un tel traitement vaccinal ; ladite première molécule ayant pour origine une première souche de *N. meningitidis* qui possède un récepteur de la transferrine humaine au moins constitué d'une sous-unité de haut poids moléculaire et d'une sous-unité de poids moléculaire moindre et dont la sous-unité de poids moléculaire moindre est reconnue par un antisérum anti-récepteur de la souche *N. meningitidis* 2394 (récepteur 2394) et n'est pas reconnue par un antisérum anti-récepteur de la souche de *N. meningitidis* 2169 (récepteur 2169) ; et ladite deuxième molécule ayant pour origine une deuxième souche de *N. meningitidis* qui possède un récepteur de la transferrine humaine au moins constitué d'une sous-unité de haut poids moléculaire et d'une sous-unité de poids moléculaire moindre et dont la sous-unité de poids moléculaire moindre est reconnue par un antisérum anti-récepteur 2169 et n'est pas reconnue par un antisérum anti-récepteur 2394.

Par "molécule capable de se lier à la transferrine humaine", on entend soit un récepteur de la transferrine humaine ayant pour origine *N. meningitidis* (c'est-à-dire une molécule comprenant notamment 2 types de sous-unités) soit uniquement la sous-unité du récepteur, capable de se lier à la transferrine humaine, ainsi qu'un fragment ou un analogue de cette sous-unité,

Un récepteur de la transferrine peut être obtenu sous forme purifiée à partir d'une souche de *N. meningitidis* préalablement cultivée dans un milieu carencé en fer sous forme libre, notamment selon la méthode de Schryvers et al, WO 90/12591, décrite de manière similaire dans Schryvers et al, Infect. Immun. (1988) 56 (5) : 1144. De manière alternative, un récepteur de la transferrine ayant pour origine une souche de *N. meningitidis* peut être produit en mettant en oeuvre les techniques du génie génétique. Le ou les fragments d'ADN codant pour les sous-unités du récepteur peuvent être exprimés conjointement ou séparément dans un système d'expression hétérologue (e.g. bactérie, levure, cellule de mammifère). Les sous-unités sous forme libre ou associées sous forme de récepteur sont dans ce cas-là recueillies à partir d'une culture et purifiées. Lorsque les sous-unités sont ainsi produites sous forme libre, on peut prévoir de les réassocier sous forme de récepteur en les soumettant à un traitement approprié.

La sous-unité capable de se lier à la transferrine humaine (sous-unité de moindre poids moléculaire) peut être obtenue sous forme purifiée (c'est-à-dire dissociée et isolée de la sous-unité de haut poids moléculaire) notamment à partir d'un récepteur purifié selon la méthode de Schryvers et al, en soumettant le récepteur à l"action d'un agent fortement dénaturant tel que l'urée 8M ou la guanidine HCl 6M, puis en séparant les sous-unités dissociées par des méthodes chromatographiques classiques telles qu'une chromatographie d'échange d'ions ou de gel de filtration. De manière alternative, la sous-unité peut être produite selon les méthodes du génie génétique. Ces méthodes sont en outre parfaitement adaptées à la production des fragments ou des analogues de la sous-unité.

A titre d'exemple, les sous-unités Tbp1 et Tbp2 des souches 2394 et 2169 sont décrites par référence à leurs séquences d'acides aminés telles que montrées dans les identificateurs de séquences n° 1 à 4 (SEQ ID N° 1 à 4).

Par "fragment de la sous-unité capable de se lier à la transferrine humaine", on signifie un peptide ayant une séquence d'acides aminés qui est incluse dans la séquence de la sous-unité. Par "analogue de la sous-unité capable de se lier à la transferrine humaine", on signifie une protéine ayant une séquence d'acides aminés qui présente un degré d'homologie d'au moins 80 %, de préférence d'au moins 90 %, de manière tout à fait préférée d'au moins 95 % avec la séquence de la sous-unité. Aux fins de la présente invention, il est bien entendu qu'un tel fragment ou un tel analogue doit conserver les propriétés immunogènes de la sous-unité.

Les souches de *N. meningitidis* 2394 (B:2a:P1.2:L2.3) et 2169 (B:9:P1.9:L3.7), communément utilisées dans les laboratoires, sont publiquement disponibles auprès de la Collection de l'Institut Pasteur, 25 rue du Dr Roux 75015 Paris sous les numéros d'enregistrement respectifs CIP 7908 et CIP 7917.

De plus, les antisérums anti-récepteur qui sont requis afin de discriminer les souches de *N. meningitidis* peuvent être obtenus comme suit :

Un récepteur est tout d'abord purifié à partir d'une souche initiale (2394 ou 2169), selon la méthode de Schryvers et al. Des lapins néozélandais albinos recoivent par voie sous-cutanée et intramusculaire 100 µg du récepteur en présence d'adjuvant complet de Freund. 21 jours et 42 jours après la première injection, les lapins recoivent à nouveau 100 µg du récepteur purifié mais ces fois-ci en présence d'adjuvant incomplet de Freund. 15 jours après la dernière injection, le sérum des animaux est prélevé, puis décomplémenté et filtré sur une membrane de porosité 0,45 µm. Le filtrat est par la suite épuisé par contact avec la souche initiale qui pour se faire, a été cultivée au préalable en présence de fer (dans ces conditions, la synthèse du récepteur de la transferrine est réprimée). Les modalités de contact sont comme suit : 10 ml du filtrat sont ajoutés à 10¹⁰ cfu (unités formant des colonies) d'une culture de la souche initiale. L'adsorption est poursuivie une nuit à 4°C, sous agitation. Les bactéries sont ensuite éliminées par centrifugation. Le surnageant est récupéré puis soumis à nouveau à 2 opérations d'adsorption successives comme précédemment décrit.

Le type d'une souche (vis-à-vis de la nature de son récepteur de la transferrine) peut être identifié à partir d'extraits membranaires derivés de cultures carencées en fer sous forme libre, en mettant en oeuvre des techniques conventionnelles telles que l'électrophorèse sur gel de SDS-PAGE, poursuivie par un immunoblotting utilisant un antisérum tel que précédemment décrit.

La première molécule entrant dans la composition vaccinale a pour origine une première souche de *N. meningitidis* qui possède un récepteur de la transferrine essentiellement constitué (i) d'une sous-unité de haut poids moléculaire, de manière avantageuse de 90 à 100 kD, de préférence de 93-95 kD environ et (ii) d'une sous-unité de moindre poids moléculaire, de manière avantageuse de 60 à 75 kD, de préférence de 65 à 72 kD, et de manière tout à fait préférée respectivement (i) de 93 kD et (ii) de 67-70 kD environ.

La deuxième molécule entrant dans la composition vaccinale a pour origine une deuxième souche de *N. meningitidis* qui possède un récepteur de la transferrine essentiellement constitué (i) d'une sous-unité de haut poids moléculaire, de manière avantageuse de 90 à 100 kD, de préférence de 95 à 100 kD, de manière tout à fait préférée de 98 kD environ et (ii) d'une sous-unité de moindre poids moléculaire, de manière avantageuse de 80 à 90 kD, de préférence de 85 à 87 kD, de manière tout à fait préférée de 87 kD environ.

Les poids moléculaires indiqués ci-avant sont des poids moléculaires apparents tels que révélés après électrophorèse d'un récepteur purifié sur gel de SDS-PAGE. Une telle électrophorèse peut être mise en oeuvre selon la méthode de Laemmli illustrée comme suit :

On prépare tout d'abord un gel de polyacrylamide (16 cm x 20 cm x 1 mm d'épaisseur) comprenant un prégel à 5 % et un gel séparateur à 7,5 % dans du tampon d'électrophorèse (Tris 6 g/l, glycine 28,8 g/l, SDS 0,1 %).

D'autre part, à 50 µl d'une solution de récepteur purifié à 0,6 mg/ml (dans le tampon phosphate 50 mM pH 8.0 contenant du Sarkosyl à 0,05 %) sont ajoutés 50 µl de tampon échantillon (Tris-HCl 62 mM pH 6.8, SDS 2 %, β-mercaptoéthanol 5 %, glycérol 1 %, bleu de bromophénol 0,001 %). Le mélange est incubé pendant 5 min dans un bain d'eau en ébullition. 17 µl (soit 5 µg de protéine) de l'échantillon ainsi préparé sont déposés dans un puits du gel. On ajoute en parallèle, un échantillon préparé de manière similaire qui contient des marqueurs de poids moléculaire. L'électrophorèse est réalisée en tampon d'électrophorèse à 50 Volts pendant 15 heures. Le gel est fixé et coloré au bleu de Coomassie.

D'une manière générale, la première ou la deuxième molécule utile aux fins de la présente invention peut avoir pour origine une souche de *N. meningitidis* de n'importe quel sérogroupe. De manière avantageuse, la première ou la deuxième molécule a pour origine une souche de *N. meningitidis* sérogoupe B. De préférence, la première et la deuxième molécules ont respectivement pour origine une première et une deuxième souches de *N. meningitidis* sérogroupe B.

Selon un aspect de l'invention tout à fait préféré, la première molécule a pour origine la souche 2394 tandis que la deuxième molécule a pour origine la souche 2169.

Une composition pharmaceutique selon l'invention peut être fabriquée de manière conventionnelle. En particulier on associe le ou les agents thérapeutiques selon l'invention avec un diluant ou un support acceptable d'un point de vue pharmaceutique. Une composition selon l'invention peut être administrée par n'importe quelle voie conventionnelle en usage dans le domaine des vaccins, en particulier par voie sous-cutanée, par voie intra-musculaire ou par voie intra-veineuse, par exemple sous forme de suspension injectable. L'administration peut avoir lieu en dose unique ou répétée une ou plusieurs fois après un certain délai d'intervalle. Le dosage approprié varie en fonction de divers paramètres, par exemple, de l'individu traité ou du mode d'administration.

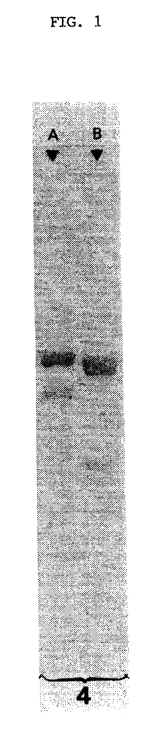

L'invention est décrite plus en détails dans les exemples ci-après et par référence à la Figure 1, qui représente une électrophorèse en gel SDS-PAGE en polyacrylamide 7,5 % dans laquelle les colonnes A et B correspondent aux récepteurs des souches *N. meningitidis* 2169 et 2394, respectivement. Les flèches à l'horizontale indiquent l'emplacement des protéines témoins de masse moléculaire apparente connue (94 kD, phosphorilase B ; 67 kD, albumine).

### EXEMPLE 1 : Purification du récepteur de la transferrine à partir de la souche 2394

1A - **Culture**
   Un lyophilisat de la souche *N. meningitidis* 2394 est repris dans environ 1 ml de bouillon Mueller-Hinton (BMH, Difco). La suspension bactérienne est ensuite étalée sur le milieu solide Muller-Hinton contenant du sang cuit (5 %).
   Après 24 h d'incubation à 37°C dans une atmosphère contenant 10 % de CO₂, la nappe bactérienne est recueillie pour ensemencer 150 ml de BMH pH 7.2, répartis en 3 erlens de 250 ml. L'incubation est poursuivie pendant 3 h à 37°C sous agitation. Chacune des 3 cultures ainsi réalisées permet d'ensemencer 400 ml de BMH pH 7,2 supplémentés avec 30 µm d'éthylènediamine - di (O-hydroxyphenyl - acetic acid) (EDDA, Sigma), qui est un agent chélatant du fer sous forme libre.
   Après 16 h de culture à 37°C sous agitation, les cultures sont contrôlées pour leur pureté par observation au microscope après une coloration de Gram. La suspension est centrifugée, le culot contenant les germes est pesé et conservé à -20°C.
1B - **Purification**
   La méthode de purification est essentiellement telle que décrite par Schryvers et al (supra).
   Le culot bactérien obtenu en 1A est décongelé, puis remis en suspension dans 200 ml de tampon Tris HCl 50 mM, pH 8.0 (tampon A). La suspension est centrifugée pendant 20 min à 15 000 xg à 4°C.Le culot est récupéré, puis remis en suspension dans du tampon A à la concentration finale de 150 g/l. Des fractions de 150 ml sont traitées pendant 8 min à 800 bars dans un lyseur de cellules travaillant sous haute pression (Rannie, modèle 8.30H). Le lysat cellulaire ainsi obtenu est centrifugé pendant 15 min à 4°C à 15 000 xg. Le surntageant est récupéré, puis centrifugé pendant 75 min à 4°C à 200 000 xg. Après élimination du surnageant, le culot est repris dans du tampon A et après dosage de protéines selon Lowry, la concentration de la suspension est ajustée à 5 mg/ml.
   A 1,4 ml de la suspension de membranes on ajoute 1,75 mg de transferrine humaine biotynylée selon le procédé décrit par Schryvers. La concentration finale de la fraction membranaire est de 4 mg/ml. Le mélange est incubé 1 heure à 37°C puis centrifugé à 100 000 xg pendant 75 min à 4°C. Le culot de membranes est repris par le tampon A contenant du NaCl 0,1M et incubé pendant 60 min à température ambiante.
   Après solubilisation, on ajoute à cette suspension un certain volume de N Lauroyl Sarkosine à 30 % (p/v) et d'EDTA 500 mM de façon que les concentrations finales en Sarkosyl et EDTA soient de 0.5 % et 5 mM respectivement. Après une incubation de 15 min à 37°C sous agitation, on ajoute 1 ml de résine streptavidine-agarose (Pierce) préalablement lavée en tampon A. La suspension est incubée 15 min à température ambiante puis centrifugée à 1 000 xg pendant 10 min. La résine est ensuite conditionnée dans une colonne et l'éluat direct est éliminé.
   La résine est lavée par 3 volumes de colonnes de tampon Tris-HCl 50 mM pH 8.0 contenant NaCl 1M, EDTA 10 mM Sarkosyl 0,5 % (tampon B) puis par un volume de colonne de tampon B contenant de la guanidine HCl 750 mM. Le récepteur de la transferrine est ensuite élué par le tampon B contenant de la guanidine HCl 2M Sarkosyl 0,05 %. L'éluat est collecté en fraction dont le volume correspond à 1 Vol., dans des tubes contenant 1 Vol. de Tris HCl 50 mM pH 8.0, NaCl 1M. La densité optique à 280 nm de l'éluat est mesurée en sortie de colonne à l'aide d'un détecteur UV.
   Les fractions correspondant au pic d'élution sont recueillies, dialysées contre du tampon phosphate 10 mM, pH 8.0 contenant du Sarkosyl 0,05 % et lyophilisées. Le lyophilisat est repris dans de l'eau à une concentration 10 fois supérieure. La solution est dialysée une seconde fois contre du tampon phosphate 50 mM pH 8.0 contenant du Sarkosyl 0,05 % (tampon C) puis la solution est filtrée sur une membrane de porosité 0,22 µm.
   Le contenu en protéines est déterminé et ajusté à 1 mg/ml par addition de tampon C, sous conditions aseptiques. Cette préparation est conservée à -70°C.

### EXEMPLE 2 : Purification du récepteur de la transferrine à partir de la souche 2169.

La culture de la souche 2169 et la purification du récepteur de la transferrine sont effectuées dans des conditions identiques à celles décrites dans l'Exemple 1.

### EXEMPLE 3 : Composition pharmaceutique vaccinale destinée à prévenir des infections à N. meningitidis.

Les solutions stériles obtenues dans les Exemples 1 et 2 sont décongelées. Afin de préparer un litre de vaccin renfermant 100 µg/ml de chacun des principes actifs, on mélange stérilement les solutions suivantes :
- Solution de récepteur 2394 à 1 mg/ml dans du tampon C 100 ml
- Solution de récepteur 2169 à 1 mg/ml dans du tampon C 100 ml
- Eau physiologique tamponnée (PBS) à pH 6.0 300 ml
- Hydroxyde d'aluminium à 10 mg Al⁺⁺⁺/ml 50 ml
- Merthiolate à 1 % (p/v) dans du PBS 10 ml
- PBS qsp 1000 ml

### EXEMPLE 4 : Mise en évidence de l'importance de la sous-unité de moindre poids moléculaire à titre d'agent vaccinal.

Des lapins néozélandais albinos recoivent par voie sous-cutanée et intramusculaire 100 µg du récepteur 2394 ou 2169 (tel que obtenu dans l'Exemple 1 ou 2) en présence d'adjuvant complet de Freund. 21 jours et 42 jours après la première injection, les lapins recoivent à nouveau 100 µg du récepteur purifié mais ces fois-ci en présence d'adjuvant incomplet de Freund. 15 jours après la dernière injection, le sérum de animaux est prélevé, puis décomplémenté et filtré sur une membrane de porosité 0,45 µm. Le filtrat est par la suite épuisé par contact avec la souche initiale (2394 ou 2169) qui pour se faire, a été cultivée au préalable en présence de fer sous forme libre (dans ces conditions, la synthèse du récepteur de la transferrine est réprimée). Les modalités de contact sont comme suit : 10 ml du filtrat sont ajoutés à 10¹⁰ cfu (unités formant des colonies) d'une culture de la souche initiale. L'adsorption est poursuivie une nuit à 4°C, sous agitation. Les bactéries sont ensuite éliminées par centrifugation. Le surnageant est récupéré puis soumis à nouveau à 2 opérations d'adsorption successives comme précédemment décrit.

Une gamme de dilution de chacun des antisérums anti-récepteur 2394 et anti-récepteur 2169 est préparée en milieu M199 (Gibco). 200 µl de chaque dilution sont déposés dans les puits d'une macroplaque de titrage (8x12in.). Un essai témoin est réalisé avec 200 µl de milieu M199. Dans chacuns des puits on ajoute (i) 100 µl d'une culture en phase de croissance exponentielle d'une souche de *N. meningitidis,* en milieu Mueller-Hinton complémenté à 30 µM EDDA et (ii) 100 µl de complément (sérum de jeune lapin dilué).

Après 30 min d'incubation à 37°C sous agitation douce, on ajoute dans chaque puits, 1 ml de milieu Mueller-Hinton contenant 1 ml d'agar noble en surfusion. Après solidification du milieu, l'incubation est poursuivie 18-24 hrs à 37°C ; puis le nombre d'unités formant des colonies dans chaque puits est évalué. L'inverse de la dernière dilution d'antisérum en présence de laquelle on observe 50 % de lyse par rapport au témoin, correspond au titre bactéricide.

Les résultats sont présentés dans le Tableau III ci-dessous :

| | Activité Bactéricide | | | |
|---|---|---|---|---|
| | **Lapin n° 1** | | **Lapin n° 2** | |
| | Sérum avant immunisation 2394 | Antisérum anti-récepteur | Sérum avant immunisation 2169 | Antisérum anti-récepteur |
| 2394 | < 8 | 2048 | < 8 | < 8 |
| 2228 | < 8 | 1024 | < 8 | < 8 |
| 2154 | < 8 | 2048 | < 8 | < 8 |
| 2234 | < 8 | 2048 | < 8 | < 8 |
| 2448 | < 8 | 256 | < 8 | < 4 |
| 2169 | < 16 | < 16 | < 8 | 1024 |
| 896 | < 8 | < 8 | < 8 | 65 |

L'antisérum anti-récepteur 2394 a une activité bactéricide uniquement à l'encontre des souches du premier type tel que défini dans la présente demande (2394, 2228, 2154, 2234 et 2448) tandis que l'antisérum anti-récepteur 2169 a une activité bactéricide uniquement à l'encontre des souches du second type (2169 et 876) Ceci suggère fortement que la production d'anticorps neutralisants est essentiellement induite par la sous-unité de moindre poids moléculaire qui porte la variabilité antigénique.

SEQ ID NO : 1

Objet : Séquence d'acides aminés de la sous-unité Tbp2 N. meningitidis 2394.

Cys Leu Gly Gly Gly Gly Ser Phe Asp Leu Asp Ser Val Glu Thr
1               5                   10                  15

Val Gln Asp Met His Ser Lys Pro Lys Tyr Glu Asp Glu Lys Ser
                20                  25                  30

Gln Pro Glu Ser Gln Gln Asp Val Ser Glu Asn Ser Gly Ala Ala
                35                  40                  45

Tyr Gly Phe Ala Val Lys Leu Pro Arg Arg Asn Ala His Phe Asn
                50                  55                  60

Pro Lys Tyr Lys Glu Lys His Lys Pro Leu Gly Ser Met Asp Trp
                65                  70                  75

Lys Lys Leu Gln Arg Gly Glu Pro Asn Ser Phe Ser Glu Arg Asp
                80                  85                  90

Glu Leu Glu Lys Lys Arg Gly Ser Ser Glu Leu Ile Glu Ser Lys
                95                  100                 105

Trp Glu Asp Gly Gln Ser Arg Val Val Gly Tyr Thr Asn Phe Thr
                110                 115                 120

Tyr Val Arg Ser Gly Tyr Val Tyr Leu Asn Lys Asn Asn Ile Asp
                125                 130                 135

Ile Lys Asn Asn Ile Val Leu Phe Gly Pro Asp Gly Tyr Leu Tyr
                140                 145                 150

Tyr Lys Gly Lys Glu Pro Ser Lys Glu Leu Pro Ser Glu Lys Ile
                155                 160                 165

Thr Tyr Lys Gly Thr Trp Asp Tyr Val Thr Asp Ala Met Glu Lys
                170                 175                 180

Gln Arg Phe Glu Gly Leu Gly Ser Ala Ala Gly Gly Asp Lys Ser
                185                 190                 195

Gly Ala Leu Ser Ala Leu Glu Glu Gly Val Leu Arg Asn Gln Ala
                200                 205                 210

Glu Ala Ser Ser Gly His Thr Asp Phe Gly Met Thr Ser Glu Phe
                215                 220                 225

Glu Val Asp Phe Ser Asp Lys Thr Ile Lys Gly Thr Leu Tyr Arg
                230                 235                 240

Asn Asn Arg Ile Thr Gln Asn Asn Ser Glu Asn Lys Gln Ile Lys
                245                 250                 255

Thr Thr Arg Tyr Thr Ile Gln Ala THr Leu His Gly Asn Arg Phe
                260                 265                 270

Lys Gly Lys Ala Leu Ala Ala Asp Lys Gly Ala Thr Asn Gly Ser
                275                 280                 285

His Pro Phe Ile Ser Asp Ser Asp Ser Leu Glu Gly Gly Phe Tyr
                290                 295                 300

Gly Pro Lys Gly Glu Glu Leu Ala Gly Lys Phe Leu Ser Asn Asp
                305                 310                 315

Asn Lys Val Ala Ala Val Phe Gly Ala Lys Gln Lys Asp Lys Lys
                320                 325                 330

Asp Gly Glu Asn Ala Ala Gly Pro Ala Thr Glu Thr Val Ile Asp
                335                 340                 345

Ala Tyr Arg Ile Thr Gly Glu Glu Phe Lys Lys Glu Gln Ile Asp
                350                 355                 360

Ser Phe Gly Asp Val Lys Lys Leu Leu Val Asp Gly Val Glu Leu
                365                 370                 375

Ser Leu Leu Pro Ser Glu Gly Asn Lys Ala Ala Phe Gln His Glu
                380                 385                 390

Ile Glu Gln Asn Gly Val Lys Ala Thr Val Cys Cys Ser Asn Leu
                395                 400                 405

Asp Tyr Met Ser Phe Gly Lys Leu Ser Lys Gku Asn Lys Asp Asp
410 415 420

Met Phe Leu Gln Gly Val Arg Thr Pro Val Ser Asp Val Ala Ala
425 430 435

Arg Thr Glu Ala Lys Tyr Arg Gly Thr Gly Thr Trp Tyr Gly Tyr
440 445 450

Ile Ala Asn Gly Thr Ser Trp Ser Gly Glu Ala Ser Asn Gln Glu
455 460 465

Gly Gly Asn Arg Ala Glu Phe Asp Val Asp Phe Ser Thr Lys Lys
470 475 480

Ile Ser Gly Thr Leu Thr Ala Lys Asp Arg Thr Ser Pro Ala Phe
485 490 495

Thr Ile Thr Ala Met Ile Lys Asp Asn Gly Phe Ser Gly Val Ala
500 505 510

Lys Thr Gly Glu Asn Gly Phe Ala Leu Asp Pro Gln Asn Thr Gly
515 520 525

Asn Ser His Tyr Thr His Ile Glu Ala Thr Val Ser Gly Gly Phe
530 535 540

Tyr Gly Lys Asn Ala Ile Glu Met Gly Gly Ser Phe Ser Phe Pro
545 550 555

Gly Asn Ala Pro Glu Gly Lys Gln Glu Lys Ala Ser Val Val Phe
560 565 570

Gly Ala Lys Arg Gln Gln Leu Val Gln
575

SEQ ID NO : 2

Objet : Séquence d'acides aminés de la sous-unité Tbp1 de N. meningitidis 2394.

Glu Asn Val Gln Ala Glu
1               5

Gln Ala Gln Glu Lys Gln Leu Asp Thr Ile Gln Val Lys Ala Lys
            10                  15                  20

Lys Gln Lys Thr Arg Arg Asp Asn Glu Val Thr Gly Leu Gly Lys
            25                  30                  35

Leu Val Lys Ser Ser Asp Thr Leu Ser Lys Glu Gln Val Leu Asn
            40                  45                  50

Ile Arg Asp Leu Thr Arg Tyr Asp Pro Gly Ile Ala Val Val Glu
            55                  60                  65

Gln Gly Arg Gly Ala Ser Ser Gly Tyr Ser Ile Arg Gly Met Asp
            70                  75                  80

Lys Asn Arg Val Ser Leu Thr Val Asp Gly Val Ser Gln Ile Gln
            85                  90                  95

Ser Tyr Thr Ala Gln Ala Ala Leu Gly Gly Thr Arg Thr Ala Gly
            100                 105                 110

Ser Ser Gly Ala Ile Asn Glu Ile Glu Tyr Glu Asn Val Lys Ala
            115                 120                 125

Val Glu Ile Ser Lys Gly Ser Asn Ser Ser Glu Tyr Gly Asn Gly
            130                 135                 140

Ala Leu Ala Gly Ser Val Ala Phe Gln Thr Lys Thr Ala Ala Asp
            145                 150                 155

Ile Ile Gly Glu Gly Lys Gln Trp Gly Ile Gln Ser Lys Thr Ala
            160                 165                 170

Tyr Ser Gly Lys Asp His Ala Leu Thr Gln Ser Leu Ala Leu Ala
            175                 180                 185

Gly Arg Ser Gly Gly Ala Glu Ala Leu Leu Ile Tyr Thr Lys Arg
            190                 195                 200

Arg Gly Arg Glu Ile His Ala His Lys Asp Ala Gly Lys Gly Val
            205                 210                 215

Gln Ser Phe Asn Arg Leu Val Leu Asp Glu Asp Lys Lys Glu Gly
            220                 225                 230

Gly Ser Gln Tyr Arg Tyr Phe Ile Val Glu Glu Glu Cys His Asn
            235                 240                 245

Gly Tyr Ala Ala Cys Lys Asn Lys Leu Lys Glu Asp Ala Ser Val
            250                 255                 260

Lys Asp Glu Arg Lys Thr Val Ser Thr Gln Asp Tyr Thr Gly Ser
            265                 270                 275

Asn Arg Leu Leu Ala Asn Pro Leu Glu Tyr Gly Ser Gln Ser Trp
            280                 285                 290

Leu Phe Arg Pro Gly Trp His Leu Asp Asn Arg His Tyr Val Gly
            295                 300                 305

Ala Val Leu Glu Arg Thr Gln Gln Thr Phe Asp Thr Arg Asp Met
            310                 315                 320

Thr Val Pro Ala Tyr Phe Thr Ser Glu Asp Tyr Val Pro Gly Ser
            325                 330                 335

Leu Lys Gly Leu Gly Lys Tyr Ser Gly Asp Asn Lys Ala Glu Arg
            340                 345                 350

Leu Phe Val Gln Gly Glu Gly Ser Thr Leu Gln Gly Ile Gly Tyr
            355                 360                 365

Gly Thr Gly Val Phe Tyr Asp Glu Arg His Thr Lys Asn Arg Tyr
            370                 375                 380

Gly Val Glu Tyr Val Tyr His Asn Ala Asp Lys Asp Thr Trp Ala
            385                 390                 395

Asp Tyr Ala Arg Leu Ser Tyr Asp Arg Gln Gly Ile Asp Leu Asp
            400                 405                 410

Asn Arg Leu Gln Gln Thr His Cys Ser His Asp Gly Ser Asp Lys
            415                 420                 425

Asn Cys Arg Pro Asp Gly Asn Lys Pro Tyr Ser Phe Tyr Lys Ser
            430                 435                 440

Asp Arg Met Ile Tyr Glu Glu Ser Arg Asn Leu Phe Gln Ala Val
            445                 450                 455

Phe Lys Lys Ala Phe Asp Thr Ala Lys Ile Arg His Asn Leu Ser
            460                 465                 470

Ile Asn Leu Gly Tyr Asp Arg Phe Lys Ser Gln Leu Ser His Ser
            475                 480                 485

Asp Tyr Tyr Leu Gln Asn Ala Val Gln Ala Tyr Asp Leu Ile Thr
            490                 495                 500

Pro Lys Lys Pro Pro Phe Pro Asn Gly Ser Lys Asp Asn Pro Tyr
            505                 510                 515

Arg Val Ser Ile Gly Lys Thr Thr Val Asn Thr Ser Pro Ile Cys
            520                 525                 530

Arg Phe Gly Asn Asn Thr Tyr Thr Asp Cys Thr Pro Arg Asn Ile
            535                 540                 545

Gly Gly Asn Gly Tyr Tyr Ala Ala Val Gln Asp Asn Val Arg Leu
            550                 555                 560

Gly Arg Trp Ala Asp Val Gly Ala Gly Ile Arg Tyr Asp Tyr Arg
            565                 570                 575

Ser Thr His Ser Glu Asp Lys Ser Val Ser Thr Gly Thr His Arg
            580                 585                 590

Asn Leu Ser Trp Asn Ala Gly Val Val Leu Lys Pro Phe Thr Trp
            595                 600                 605

Met Asp Leu Thr Tyr Arg Ala Ser Thr Gly Phe Arg Leu Pro Ser
            610                 615                 620

Phe Ala Glu Met Tyr Gly Trp Arg Ala Gly Glu Ser Leu Lys Thr
            625                 630                 635

Leu Asp Leu Lys Pro Glu Lys Ser Phe Asn Arg Glu Ala Gly Ile
            640                 645                 650

Val Phe Lys Gly Asp Phe Gly Asn Leu Glu Ala Ser Tyr Phe Asn
            655                 660                 665

Asn Ala Tyr Arg Asp Leu Ile Ala Phe Gly Tyr Glu Thr Arg Thr
            670                 675                 680

Gln Asn Gly Gln Thr Ser Ala Ser Gly Asp Pro Gly Tyr Arg Asn
            685                 690                 695

Ala Gln Asn Ala Arg Ile Ala Gly Ile Asn Ile Leu Gly Lys Ile
            700                 705                 710

Asp Trp His Gly Val Trp Gly Gly Leu Pro Asp Gly Leu Tyr Ser
            715                 720                 725

Thr Leu Ala Tyr Asn Arg Ile Lys Val Lys Asp Ala Asp Ile Arg
            730                 735                 740

Ala Asp Arg Thr Phe Val Thr Ser Tyr Leu Phe Asp Ala Val Gln
            745                 750                 755

Pro Ser Arg Tyr Val Leu Gly Leu Gly Tyr Asp His Pro Asp Gly
            760                 765                 770

Ile Trp Gly Ile Asn Thr Met Phe Thr Tyr Ser Lys Ala Lys Ser
            775                 780                 785

Val Asp Glu Leu Leu Gly Ser Gln Ala Leu Leu Asn Gly Asn Ala
            790                 795                 800

Asn Ala Lys Lys Ala Ala Ser Arg Arg Thr Arg Pro Trp Tyr Val
            805                 810                 815

Thr Asp Val Ser Gly Tyr Tyr Asn Ile Lys Lys His Leu Thr Leu
            820                 825                 830

Arg Ala Gly Val Tyr Asn Leu Leu Asn Tyr Arg Tyr Val Thr Trp
            835                 840                 845

Glu Asn Val Arg Gln Thr Ala Gly Gly Ala Val Asn Gln His Lys
            850                 855                 860

Asn Val Gly Val Tyr Asn Arg Tyr Ala Ala Pro Gly Arg Asn Tyr
            865                 870                 875

Thr Phe Ser Leu Glu Met Lys Phe
            880

SEQ ID NO : 3

Objet : Séquence d'acides aminés de la sous-unité Tbp1 de N. meningitidis 2169.

Glu Asn Val Gln Ala Gly
1               5

Gln Ala Gln Glu Lys Gln Leu Asp Thr Ile Gln Val Lys Ala Lys
            10                  15                  20

Lys Gln Lys Thr Arg Arg Asp Asn Glu Val Thr Gly Leu Gly Lys
            25                  30                  35

Leu Val Lys Thr Ala Asp Thr Leu Ser Lys Glu Gln Val Leu Asp
            40                  45                  50

Ile Arg Asp Leu Thr Arg Tyr Asp Pro Gly Ile Ala Val Val Glu
            55                  60                  65

Gln Gly Arg Gly Ala Ser Ser Gly Tyr Ser Ile Arg Gly Met Asp
            70                  75                  80

Lys Asn Arg Val Ser Leu Thr Val Asp Gly Leu Ala Gln Ile Gln
            85                  90                  95

Ser Tyr Thr Ala Gln Ala Ala Leu Gly Gly Thr Arg Thr Ala Gly
            100                 105                 110

Ser Ser Gly Ala Ile Asn Glu Ile Glu Tyr Glu Asn Val Lys Ala
            115                 120                 125

Val Glu Ile Ser Lys Gly Ser Asn Ser Val Glu Gln Gly Ser Gly
            130                 135                 140

Ala Leu Ala Gly Ser Val Ala Phe Gln Tyr Lys Thr Ala Asp Asp
            145                 150                 155

Val Ile Gly Glu Gly Arg Gln Trp Gly Ile Gln Ser Lys Thr Ala
            160                 165                 170

Tyr Ser Gly Lys Asn Arg Gly Leu Thr Gln Ser Ile Ala Leu Ala
            175                 180                 185

Gly Arg Ile Gly Gly Ala Glu Ala Leu Leu Ile His Thr Gly Arg
            190                 195                 200

Arg Ala Gly Glu Ile Arg Ala His Glu Asp Ala Gly Arg Gly Val
            205                 210                 215

Gln Ser Phe Asn Arg Leu Val Pro Val Glu Asp Ser Ser Glu Tyr
            220                 225                 230

Ala Tyr Phe Ile Val Glu Asp Glu Cys Glu Gly Lys Asn Tyr Glu
            235                 240                 245

Thr Cys Lys Ser Lys Pro Lys Lys ASp Val Val Gly Lys Asp Glu
            250                 255                 260

Arg Gln Thr Val Ser Thr Arg Asp Tyr Thr Gly Pro Asn Arg Phe
            265                 270                 275

Leu Ala Asp Pro Leu Ser Tyr Glu Ser Arg Ser Trp Leu Phe Arg
            280                 285                 290

Pro Gly Phe Arg Phe Glu Asn Lys Arg His Tyr Ile Gly Gly Ile
            295                 300                 305

Leu Glu His Thr Gln Gln Thr Phe Asp Thr Arg Asp Met Thr Val
            310                 315                 320

Pro Ala Phe Leu Thr Lys Ala Val Phe Asp Ala Asn Ser Lys Gln
            325                 330                 335

Ala Gly Ser Leu Pro Gly Asn Gly Lys Tyr Ala Gly Asn His Lys
            340                 345                 350

Tyr Gly Gly Leu Phe Thr Asn Gly Glu Asn Gly Ala Leu Val Gly
            355                 360                 365

Ala Glu Tyr Gly Thr Gly Val Phe Tyr Asp Glu Thr His Thr Lys
            370                 375                 380

Ser Arg Tyr Gly Leu Glu Tyr Val Tyr Thr Asn Ala Asp Lys Asp
            385                 390                 395

Thr Trp Ala Asp Tyr Ala Arg Leu Ser Tyr Asp Arg Gln Gly Ile
            400                 405                 410

Gly Leu Asp Asn His Phe Gln Gln Thr His Cys Ser Ala Asp Gly
            415                 420                 425

Ser Asp Lys Tyr Cys Arg Pro Ser Ala Asp Lys Pro Phe Ser Tyr
            430                 435                 440

Tyr Lys Ser Asp Arg Val Ile Tyr Gly Glu Ser His Arg Leu Leu
            445                 450                 455

Gln Ala Ala Phe Lys Lys Ser Phe Asp Thr Ala Lys Ile Arg His
            460                 465                 470

Asn Leu Ser Val Asn Leu Gly Phe Asp Arg Phe Asp Ser Asn Leu
            475                 480                 485

Arg His Gln Asp Tyr Tyr Tyr Gln His Ala Asn Arg Ala Tyr Ser
            490                 495                 500

Ser Lys Thr Pro Pro Lys Thr Ala Asn Pro Asn Gly Asp Lys Ser
            505                 510                 515

Lys Pro Tyr Trp Val Ser Ile Gly Gly Gly Asn Val Val Thr Gly
            520                 525                 530

Gln Ile Cys Leu Phe Gly Asn Asn Thr Tyr Thr Asp Cys Thr Pro
            535                 540                 545

Arg Ser Ile Asn Gly Lys Ser Tyr Tyr Ala Ala Val Arg Asp Asn
            550                 555                 560

Val Arg Leu Gly Arg Trp Ala Asp Val Gly Ala Gly Leu Arg Tyr
            565                 570                 575

Asp Tyr Arg Ser Thr His Ser Asp Asp Gly Ser Val Ser Thr Gly
            580                 585                 590

Thr His Arg Thr Leu Ser Trp Asn Ala Gly Ile Val Leu Lys Pro
            595                 600                 605

Ala Asp Trp Leu Asp Leu Thr Tyr Arg Thr Ser Thr Gly Phe Arg
            610                 615                 620

Leu Pro Ser Phe Ala Glu Met Tyr Gly Trp Arg Ser Gly Val Gln
            625                 630                 635

Ser Lys Ala Val Lys Ile Asp Pro Glu Lys Ser Phe Asn Lys Glu
            640                 645                 650

Ala Gly Ile Val Phe Lys Gly Asp Phe Gly Asn Leu Glu Ala Ser
            655                 660                 665

Trp Phe Asn Asn Ala Tyr Arg Asp Leu Ile Val Arg Gly Tyr Glu
            670                 675                 680

Ala Gln Ile Lys Asn Gly Lys Glu Glu Ala Lys Gly Asp Pro Ala
            685                 690                 695

Tyr Leu Asn Ala Gln Ser Ala Arg Ile Thr Gly Ile Asn Ile Leu
            700                 705                 710

Gly Lys Ile Asp Trp Asn Gly Val Trp Asp Lys Leu Pro Glu Gly
            715                 720                 725

Trp Tyr Ser Thr Phe Ala Tyr Asn Arg Val His Val Arg Asp Ile
            730                 735                 740

Lys Lys Arg Ala Asp Arg Thr Asp Ile Gln Ser His Leu Phe Asp
            745                 750                 755

Ala Ile Gln Pro Ser Arg Tyr Val Val Gly Leu Gly Tyr Asp Gln
            760                 765                 770

Pro Glu Gly Lys Trp Gly Val Asn Gly Met Leu Thr Tyr Ser Lys
            775                 780                 785

Ala Lys Glu Ile Thr Glu Leu Leu Gly Ser Arg Ala Leu Leu Asn
            790                 795                 800

Gly Asn Ser Arg Asn Thr Lys Ala Thr Ala Arg Arg Thr Arg Pro
            805                 810                 815

Trp Tyr Ile Val Asp Val Ser Gly Tyr Tyr Thr Ile Lys Lys His
            820                 825                 830

Phe Thr Leu Arg Ala Gly Val Tyr Asn Leu Leu Asn Tyr Arg Tyr
            835                 840                 845

Val Thr Trp Glu Asn Val Arg Gln Thr Ala Gly Gly Ala Val Asn
            850                 855                 860

Gln His Lys Asn Val Gly Val Tyr Asn Arg Tyr Ala Ala Pro Gly
            865                 870                 875

Arg Asn Tyr Thr Phe Ser Leu Glu Met Lys Phe
            880                 885

SEQ ID NO : 4

Objet : Sequence d'acides aminés de la sous-unité Tbp2 de N. meningitidis 2169.

Cys Leu Gly Gly Gly Gly Ser Phe Asp Leu
1               5                   10

Asp Ser Val Asp Thr Glu Ala Pro Arg Pro Ala Pro Lys Tyr Gln
                15                  20                  25

Asp Val Ser Ser Glu Lys Pro Gln Ala Gln Lys Asp Gln Gly Gly
                30                  35                  40

Tyr Gly Phe Ala Met Arg Leu Lys Arg Arg Asn Trp Tyr Pro Gly
                45                  50                  55

Ala Glu Glu Ser Glu Val Lys Leu Asn Glu Ser Asp Trp Glu Ala
                60                  65                  70

Thr Gly Leu Pro Thr Lys Pro Lys Glu Leu Pro Lys Arg Gln Lys
                75                  80                  85

Ser Val Ile Glu Lys Val Glu Thr Asp Gly Asp Ser Asp Ile Tyr
                90                  95                  100

Ser Ser Pro Tyr Leu Thr Pro Ser Asn His Gln Asn Gly Ser Ala
                105                 110                 115

Gly Asn Gly Val Asn Gln Pro Lys Asn Gln Ala Thr Gly His Glu
                120                 125                 130

Asn Phe Gln Tyr Val Tyr Ser Gly Trp Phe Tyr Lys His Ala Ala
                135                 140                 145

Ser Glu Lys Asp Phe Ser Asn Lys Lys Ile Lys Ser Gly Asp Asp
                150                 155                 160

Gly Tyr Ile Phe Tyr His Gly Glu Lys Pro Ser Arg Gln Leu Pro
                165                 170                 175

Ala Ser Gly Lys Val Ile Tyr Lys Gly Val Trp His Phe Val Thr
                180                 185                 190

Asp Thr Lys Lys Gly Gln Asp Phe Arg Glu Ile Ile Gln Pro Ser
                195                 200                 205

Lys Lys Gln Gly Asp Arg Tyr Ser Gly Phe Ser Gly Asp Gly Ser
                210                 215                 220

Glu Glu Tyr Ser Asn Lys Asn Glu Ser Thr Leu Lys Asp Asp His
                225                 230                 235

Glu Gly Tyr Gly Phe Thr Ser Asn Leu Glu Val Asp Phe Gly Asn
                240                 245                 250

Lys Lys Leu Thr Gly Lys Leu Ile Arg Asn Asn Ala Ser Leu Asn
                255                 260                 265

Asn Asn Thr Asn Asn Asp Lys His Thr Thr Gln Tyr Tyr Ser Leu
                    270                 275                 280

Asp Ala Gln Ile Thr Gly Asn Arg Phe Asn Gly Thr Ala Thr Ala
                    285                 290                 295

Thr Asp Lys Lys Glu Asn Glu Thr Lys Leu His Pro Phe Val Ser
                    300                 305                 310

Asp Ser Ser Ser Leu Ser Gly Gly Phe Phe Gly Pro Gln Gly Glu
                    315                 320                 325

Glu Leu Gly Phe Arg Phe Leu Ser Asp Asp Gln Lys Val Ala Val
                    330                 335                 340

Val Gly Ser Ala Lys Thr Lys Asp Lys Leu Glu Asn Gly Ala Ala
                    345                 350                 355

Ala Ser Gly Ser Thr Gly Ala Ala Ala Ser Gly Gly Ala Ala Gly
                    360                 365                 370

Thr Ser Ser Glu Asn Ser Lys Leu Thr Thr Val Leu Asp Ala Val
                    375                 380                 385

Glu Leu Thr Leu Asn Asp Lys Lys Ile Lys Asn Leu Asp Asn Phe
                    390                 395                 400

Ser Asn Ala Ala Gln Leu Val Val Asp Gly Ile Met Ile Pro Leu
                    405                 410                 415

Leu Pro Lys Asp Ser Glu Ser Gly Asn Thr Gln Ala Asp Lys Gly
                    420                 425                 430

Lys Asn Gly Gly Thr Glu Phe Thr Arg Lys Phe Glu His Thr Pro
                    435                 440                 445

Glu Ser Asp Lys Lys Asp Ala Gln Ala Gly Thr Gln Thr Asn Gly
                    450                 455                 460

Ala Gln Thr Ala Ser Asn Thr Ala Gly Asp Thr Asn Gly Lys Thr
                    465                 470                 475

Lys Thr Tyr Glu Val Glu Val Cys Cys Ser Asn Leu Asn Tyr Leu
                    480                 485                 490

Lys Tyr Gly Met Leu Thr Arg Lys Asn Ser Lys Ser Ala Met Gln
                    495                 500                 505

Ala Gly Gly Asn Ser Ser Gln Ala Asp Ala Lys Thr Glu Gln Val
                    510                 515                 520

Glu Gln Ser Met Phe Leu Gln Gly Glu Arg Thr Asp Glu Lys Glu
                    525                 530                 535

Ile Pro Thr Asp Gln Asn Val Val Tyr Arg Gly Ser Trp Tyr Gly
                    540                 545                 550

His Ile Ala Asn Gly Thr Ser Trp Ser Gly Asn Ala Ser Asp Lys
                    555                 560                 565

Glu Gly Gly Asn Arg Ala Glu Phe Thr Val Asn Phe Ala Asp Lys
                    570                 575                 580

Lys Ile Thr Gly Lys Leu Thr Ala Glu Asn Arg Gln Ala Gln Thr
                    585                 590                 595

Phe Thr Ile Glu Gly Met Ile Gln Gly Asn Gly Phe Glu Gly Thr
                    600                 605                 610

Ala Lys Thr Ala Glu Ser Gly Phe Asp Leu Asp Gln Lys Asn Thr
                    615                 620                 625

Thr Arg Thr Pro Lys Ala Tyr Ile Thr Asp Ala Lys Val Lys Gly
                    630                 635                 640

Gly Phe Tyr Gly Pro Lys Ala Glu Glu Leu Gly Gly Trp Phe Ala
                    645                 650                 655

Tyr Pro Gly Asp Lys Gln Thr Glu Lys Ala Thr Ala Thr Ser Ser
                    660                 665                 670

Asp Gly Asn Ser Ala Ser Ser Ala Thr Val Val Phe Gly Ala Lys
                    675                 680                 685

Arg Gln Gln Pro Val Gln
                    690

## Revendications

1. Une composition pharmaceutique vaccinale destinée à la prévention ou à l'atténuation des effets d'une infection à *Neisseria meningitidis*, qui comprend à titre d'agents thérapeutiques au moins une première et une deuxième molécules capables de se lier à la transferrine humaine ; ladite première molécule ayant pour origine une première souche de *N. meningitidis* qui possède un récepteur de la transferrine humaine au moins constitué d'une sous-unité de haut poids moléculaire et d'une sous-unité de poids moléculaire moindre et dont la sous-unité de poids moléculaire moindre est reconnue par un antisérum anti-récepteur de la souche *N. meningitidis* 2394 (récepteur 2394) et n'est pas reconnue par un antisérum anti-récepteur de la souche de *N. meningitidis* 2169 (récepteur 2169) ; et ladite deuxième molécule ayant pour origine une deuxième souche de *N. meningitidis* qui possède un récepteur de la transferrine humaine au moins constitué d'une sous-unité de haut poids moléculaire et d'une sous-unité de poids moléculaire moindre et dont la sous-unité de poids moléculaire moindre est reconnue par un antisérum anti-récepteur 2169 et n'est pas reconnue par un antisérum anti-récepteur 2394.

2. Une composition pharmaceutique vaccinale selon la revendication 1, qui comprend à titre d'agents thérapeutiques au moins une première et une deuxième molécules capables de se lier à la transferrine humaine ; ladite première molécule ayant pour origine une première souche de *N. meningitidis* qui possède un récepteur de la transferrine humaine dont la sous-unité de haut poids moléculaire et la sous-unité de poids moléculaire moindre sont reconnues par un antisérum anti-récepteur 2394 ; et ladite deuxième molécule ayant pour origine une deuxième souche de *N. meningitidis* qui possède un récepteur de la transferrine humaine dont la sous-unité de haut poids moléculaire et la sous-unité de poids moléculaire moindre sont reconnues par un antisérum anti-récepteur 2169.

3. Une composition pharmaceutique vaccinale selon la revendication 1 ou 2, qui comprend à titre d'agent thérapeutiques, au moins une première et une deuxième molécules capables de se lier à la transferrine humaine ; ladite première molécule ayant pour origine une première souche de *N. meningitidis* qui possède un récepteur de la transferrine humaine essentiellement constitué d'une sous-unité de haut poids moléculaire de 90 kD à 100 kD et d'une sous-unité de moindre poids moléculaire de 60 kD à 75 kD ; et ladite deuxième molécule ayant pour origine une deuxième souche de *N. meningitidis* qui possède un récepteur de la transferrine humaine essentiellement constitué d'une sous-unité d'un poids moléculaire élevé de 90 kD à 100 kD et d'une sous-unité d'un poids moléculaire moindre de 80 kD à 90 kD.

4. Une composition pharmaceutique vaccinale selon la revendication 3, dans laquelle ladite première molécule a pour origine une première souche de *N. meningitidis* qui possède un récepteur de la transferrine humaine essentiellement constitué d'une sous-unité de haut poids moléculaire de 93-95 kD et d'une sous-unité de moindre poids moléculaire de 65 kD à 72 kD.

5. Une composition pharmaceutique vaccinale selon la revendication 4, dans laquelle ladite première molécule a pour origine une première souche de *N. meningitidis* qui possède un récepteur de la transferrine humaine essentiellement constitué d'une sous-unité de haut poids moléculaire de 93 kD environ et d'une sous-unité de moindre poids moléculaire de 67-70 kD environ.

6. Une composition pharmaceutique vaccinale selon la revendication 5, dans laquelle ladite deuxième molécule a pour origine une deuxième souche de *N. meningitidis* qui possède un récepteur de la transferrine humaine essentiellement constitué d'une sous-unité de haut poids moléculaire de 95 kD à 100 kD et d'une sous-unité de moindre poids moléculaire de 85 kD à 87 kD.

7. Une composition pharmaceutique vaccinale selon la revendication 6, dans laquelle ladite deuxième molécule a pour origine une deuxième souche de *N. meningitidis* qui possède un récepteur de la transferrine humaine essentiellement constitué d'une sous-unité de haut poids moléculaire de 98 kD environ et d'une sous-unité d'un poids moléculaire moindre de 87 kD environ.

8. Une composition pharmaceutique vaccinale selon l'une des revendications 1 à 7, dans laquelle ladite première molécule capable de se lier à la transferrine humaine et ayant pour origine ladite première souche, est le récepteur de la transferrine humaine de ladite première souche.

9. Une composition pharmaceutique vaccinale selon l'une des revendications 1 à 7, dans laquelle ladite première molécule capable de se lier à la transferrine humaine et ayant pour origine ladite première souche, est la sous-unité de moindre poids moléculaire du récepteur de la transferrine humaine de ladite première souche, un fragment ou un analogue de ladite sous-unité de moindre poids moléculaire.

10. Une composition pharmaceutique vaccinale selon la revendication 9, dans laquelle ladite première molécule capable de se lier à la transferrine humaine et ayant pour origine ladite première souche, est la sous-unité de moindre poids moléculaire du récepteur de la transferrine humaine de ladite première souche.

11. Une composition pharmaceutique vaccinale selon l'une des revendications 1 à 10, dans laquelle ladite deuxième molécule capable de se lier à la transferrine humaine et ayant pour origine ladite deuxième souche, est le récepteur de la transferrine humaine de ladite deuxième souche.

12. Une composition pharmaceutique vaccinale selon l'une des revendications 1 à 10, dans laquelle ladite deuxième molécule capable de se lier à la transferrine humaine et ayant pour origine ladite deuxième souche, est la sous-unité de moindre poids moléculaire du récepteur de la transferrine humaine de ladite deuxième souche, un fragment ou un analogue de ladite sous-unité de moindre poids moléculaire.

13. Une composition pharmaceutique vaccinale selon la revendication 12, dans laquelle ladite deuxième molécule capable de se lier à la transferrine humaine et ayant pour origine ladite deuxième souche, est la sous-unité de moindre poids moléculaire du récepteur de la transferrine humaine de ladite deuxième souche.

14. Une composition pharmaceutique vaccinale selon l'une des revendications 1 à 13, dans laquelle lesdites première et deuxième molécules ont respectivement pour origine une première et deuxième souches de *N. meningitidis* sérogroupe B.

## Claims

1. A pharmaceutical vaccine composition intended for preventing or attenuating the effects of a *Neisseria meningitidis* infection, which contains as therapeutic agents at least a first and second molecule capable of binding to human transferrin; said first molecule originating from a first strain of *N. meningitidis* which has a human transferrin receptor consisting at least of a subunit of high molecular weight and a subunit of lower molecular weight, wherein the subunit of lower molecular weight is recognised by an anti-receptor antiserum of the strain *N. meningitidis* 2394 (receptor 2394) and is not recognised by an anti-receptor antiserum of the strain of *N. meningitidis* 2169 (receptor 2169); and said second molecule originating from a second strain of *N. meningitidis* which has a human transferrin receptor consisting at least of a subunit of high molecular weight and a subunit of lower molecular weight and wherein the subunit of lower molecular weight is recognised by an anti-receptor 2169 antiserum and is not recognised by an anti-receptor 2394 antiserum.

2. A pharmaceutical vaccine composition according to claim 1, which contains as therapeutic agents at least a first and second molecule capable of binding to human transferrin; said first molecule originating from a first strain of *N. meningitidis* which has a human transferrin receptor wherein the subunit of high molecular weight and the subunit of lower molecular weight are recognised by an anti-receptor 2394 antiserum; and said second molecule originating from a second strain of *N. meningitidis* which has a human transferrin receptor wherein the subunit of high molecular weight and the subunit of lower molecular weight are recognised by an anti-receptor 2169 antiserum.

3. A pharmaceutical vaccine composition according to claim 1 or 2, which contains as therapeutic agents at least a first and second molecule capable of binding to human transferrin; said first molecule originating from a first strain of *N. meningitidis* which has a human transferrin receptor consisting essentially of a subunit of high molecular weight of 90 kD to 100 kD and a subunit of lower molecular weight of 60 kD to 75 kD; and said second molecule originating from a second strain of *N. meningitidis* which has a human transferrin receptor consisting essentially of a subunit of high molecular weight of 90 kD to 100 kD and a subunit of lower molecular weight of 80 kD to 90 kD.

4. A pharmaceutical vaccine composition according to claim 3, wherein said first molecule originates from a first strain of *N. meningitidis* which has a human transferrin receptor consisting essentially of a subunit of high molecular weight of 93 - 95 kD and a subunit of lower molecular weight of 65 to 72 kD.

5. A pharmaceutical vaccine composition according to claim 4, wherein said first molecule originates from a first strain of *N. meningitidis* which has a human transferrin receptor consisting essentially of a subunit of high molecular weight of about 93 kD and a subunit of lower molecular weight of about 67 - 70 kD.

6. A pharmaceutical vaccine composition according to claim 5, wherein said second molecule originates from a second strain of *N. meningitidis* which has a human transferrin receptor consisting essentially of a subunit of high molecular weight of 95 to 100 kD and a subunit of lower molecular weight of 85 to 87 kD.

7. A pharmaceutical vaccine composition according to claim 6, wherein said second molecule originates from a second strain of *N. meningitidis* which has a human transferrin receptor consisting essentially of a subunit of high molecular weight of about 98 kD and a subunit of lower molecular weight of about 87 kD.

8. A pharmaceutical vaccine composition according to one of claims 1 to 7, wherein said first molecule capable of binding to human transferrin and originating from said first strain is the human transferrin receptor of said first strain.

9. A pharmaceutical vaccine composition according to one of claims 1 to 7, wherein said first molecule capable of binding to human transferrin and originating from said first strain is the subunit of lower molecular weight of the human transferrin receptor of said first strain, or a fragment or analogue of said subunit of lower molecular weight.

10. A pharmaceutical vaccine composition according to claim 9, wherein said first molecule capable of binding to human transferrin and originating from said first strain is the subunit of lower molecular weight of the human transferrin receptor of said first strain.

11. A pharmaceutical vaccine composition according to one of claims 1 to 10, wherein said second molecule capable of binding to human transferrin and originating from said second strain is the human transferrin receptor of said second strain.

12. A pharmaceutical vaccine composition according to one of claims 1 to 10, wherein said second molecule capable of binding to human transferrin and originating from said second strain is the subunit of lower molecular weight of the human transferrin receptor of said second strain, or a fragment or analogue of said subunit of lower molecular weight.

13. A pharmaceutical vaccine composition according to claim 12, wherein said second molecule capable of binding to human transferrin and originating from said second strain is the subunit of lower molecular weight of the human transferrin receptor of said second strain.

14. A pharmaceutical vaccine composition according to one of claims 1 to 13, wherein said first and second molecules originate, respectively, from a first and second strain of *N. meningitidis* serogroup B.

## Patentansprüche

1. Eine pharmazeutische Vakzinzusammensetzung zur Vorbeugung und Milderung der Wirkungen einer Infektion durch *Neisseria meningitidis*, die als therapeutische Mittel wenigstens ein erstes und ein zweites Molekül enthält, die zur Bindung an menschliches Transferrin befähigt sind, wobei das erste Molekül einem ersten Stamm von *N. meningitidis* entstammt, der einen Rezeptor für menschliches Transferrin besitzt, welcher mindestens gebildet ist aus einer hochmolekularen Untereinheit und einer Untereinheit mit geringerem Molekulargewicht, und dessen Untereinheit mit geringerem Molekulargewicht erkannt wird durch ein gegen den Rezeptor des Stamms *N. meningitidis* 2394 (Rezeptor 2394) gerichtetes Antiserum und nicht erkannt wird durch ein gegen den Rezeptor des Stamms *N. meningitides* 2169 (Rezeptor 2169) gerichtetes Antiserum und wobei das zweite Molekül einem zweiten *N. meningitidis* Stamm entstammt, der einen Rezeptor für menschliches Transferrin besitzt, welcher mindestens aus einer hochmolekularen Untereinheit und einer Untereinheit eines geringeren Molekulargewichts entstammt und dessen Untereinheit mit einem geringeren Molekulargewicht erkannt wird durch ein gegen den Rezeptor 2169 gerichtetes Antiserum und nicht erkannt wird durch ein gegen den Rezeptor 2394 gerichtetes Antiserum.

2. Eine pharmazeutische Vakzinzusammensetzung nach Anspruch 1, die als therapeutische Mittel wenigstens ein erstes und ein zweites Molekül enthält, welche zur Bindung an menschliches Transferrin befähigt sind, wobei das erste Molekül einem ersten Stamm von *N. meningitidis* entstammt, der einen Rezeptor für menschliches Transferrin besitzt, dessen hochmolekularegewichtige Untereinheit und dessen Untereinheit mit einem geringeren Molekulargewicht durch ein gegen den Rezeptor 2394 gerichtetes Antiserum erkannt werden, und wobei das zweite Molekül einem ersten Stamm von *N. meningitidis* entstammt, der einen Rezeptor für menschliches Transferrin enthält, dessen hochmolekulargewichtige Untereinheit und dessen Untereinheit mit geringerem Molekulargewicht erkannt werden durch ein gegen den Rezeptor 2169 gerichtetes Antiserum.

3. Eine pharmazeutische Vakzinzusammensetzung nach Anspruch 1 oder 2, die als therapeutische Mittel wenigstens ein erstes und ein zweites Molekül enthält, die zur Bindung an menschliches Transferrin befähigt sind, wobei das erste Molekül einem Stamm von *N. meningitidis* entstammt, der einen Rezeptor für menschliches Transferrin enthält, welches im wesentlichen aufgebaut ist aus einer hochmolekulargewichtigen Untereinheit von 90 kD bis 100 kD und einer Untereinheit mit geringerem Molekulargewicht von 60 kD bis 75 kD und wobei dieses zweite Molekül einem zweiten Stamm von *N. meningitidis* entstammt, der einen Rezeptor für menschliches Transferrin enthält, das im wesentlichen aufgebaut ist aus einer Untereinheit mit einem erhöhten Molekulargewicht von 90 kD bis 100 kD und einer Untereinheit eines geringeren Molekulargewichts von 80 kD bis 90 kD.

4. Eine pharmazeutische Vakzinzusammensetzung nach Ansprch 3, in der das erste Molekül einem Stamm von *N. meningitidis* entstammt, der einen Rezeptor für menschliches Transferrin besitzt und im wesentlichen aufgebaut ist aus einer hochmolekulargewichtigen Untereinheit von 93 bis 95 kD und einer Untereinheit mit geringerem Molekulargewicht von 65 kD bis 72 kD.

5. Eine pharmazeutische Vakzinzusammensetzung nach Anspruch 4, in der das erste Molekül einem Stamm von *N. meningitidis* entstammt, der einen Rezeptor für menschliches Transferrin besitzt und im wesentlichen aufgebaut ist aus einer hochmolekulargewichtigen Untereinheit von etwa 93 kD und einer Untereinheit mit geringerem Molekulargewicht von 67 bis etwa 70 kD.

6. Eine pharmazeutische Vakzinzusammensetzung nach Anspruch 5, in der das zweite Molekül einem Stamm von *N. meningitidis* entstammt, der einen Rezeptor für menschliches Transferrin besitzt, welcher im wesentlichen aufgebaut ist aus einer hochmolekulargewichtigen Untereinheit von 95 kD bis 100 kD und einer Untereinheit eines geringeren Molekulargewichts von 85 kD bis 87 kD.

7. Eine pharmazeutische Vakzinzusammensetzung nach Anspruch 6, in der das zweite Molekül einem zweiten Stamm von *N. meningitidis* entstammt, der einen Rezeptor für menschliches Transferrin besitzt und im wesentlichen aufgebaut ist aus einer hochmolekulargewichtigen Untereinheit von etwa 98 kD und und einer Untereinheit eines geringeren Molekulargewichts von etwa 87 kD,

8. Eine pharmazeutische Vakzinzusammensetzung nach einem der Ansprüche 1 bis 7, in der das erste Molekül, das zur Bindung an menschliches Transferrin befähigt ist, und diesem ersten Stamm entstammt, der Rezeptor für menschliches Transferrin dieses ersten Stamms ist.

9. Eine pharmazeutische Vakzinzusammensetzung nach einem der Ansprüche 1 bis 7, in der dieses erste Molekül, das zur Bindung an menschliches Transferrin befähigt ist und diesem ersten Stamm entstammt, die Untereinheit mit geringerem Molekulargewicht des Rezeptors für menschliches Transferrin dieses ersten Stamms, ein Fragment oder ein Analoges dieser Untereinheit mit geringerem Molekulargewicht ist.

10. Eine pharmazeutische Vakzinzusammensetzung nach Anspruch 9, in der das erste Molekül, das zur Bindung von menschlichem Transferrin befähigt ist und einem ersten Stamm entstammt, die Untereinheit mit geringerem Molekulargewicht des Rezeptors für menschliches Transferrin dieses ersten Stamms ist.

11. Eine pharmazeutische Vakzinzusammensetzung nach den Ansprüchen 1 bis 10, in der das zweite Molekül, das zur Bindung an menschliches Transferrin befähigt ist, und dem zweiten Stamm entstammt, der Rezeptor für menschliches Transferrin dieses zweites Stamms ist.

12. Eine pharmazeutische Vakzinzusammensetzung nach den Ansprüchen 1 bis 10, in der das zweite Molekül, das zur Bindung an menschliches Transferrin befähigt ist, und dem zweiten Stamm entstammt, die Untereinheit geringeren Molekulargewichts des Rezeptors für menschliches Transferrin dieses zweites Stamms, ein Fragment oder ein Analoges dieser Untereinheit mit geringerem Molekulargewicht ist.

13. Eine pharmazeutische Vakzinzusammensetzung nach Anspruch 12, in der das zweite Molekül, das zur Bindung an menschliches Transferrin befähigt ist und dem zweiten Stamm entstammt, die Untereinheit geringeren Molekulargewichts des Rezeptors für menschliches Transferrin dieses zweites Stamms ist.

14. Eine pharmazeutische Vakzinzusammensetzung nach einem der Ansprüche 1 bis 13, in der das erste und das zweite Molekül jeweils einem ersten und einem zweiten Stamm von *N. meningitidis* Serumgruppe B entstammen.
